# FASCICULE DE BREVET EUROPEEN

(11) **EP 1 275 387 B1**
(45) Date de publication et mention de la délivrance du brevet: **03.05.2006**
(21) Numéro de dépôt: 02018031.1
(22) Date de dépôt: 16.01.1998
(51) Int. Cl.: A61K 31/215, A61K 9/16, A61K 9/20, A61K 9/50, A61K 9/10

(54) **Procédé de préparation de compositions pharmaceutiques de fénofibrate présentant une biodisponibilité élévée**
Verfahren zur Herstellung von Fenofibrathaltigen Arzneizusammensetzungen mit erhöhter Bioverfügbarkeit
Process of manufacturing Fenofibrate pharmaceutical compositions with a higher bioavailability

(30) Priorité: 17.01.1997 FR 9700479
(43) Date de publication de la demande: 15.01.2003
(62) Demande divisionnaire de: 98900125.0
(73) Titulaire: LABORATOIRES FOURNIER S.A., 21000 Dijon (FR)
(72) Inventeur: Stamm, André, 67870 Griesheim (FR); Seth, Pawan, Irvine, CA 92612 (US)
(74) Mandataire: Hirsch & Associés

(56) Documents cités:
- EP-A1- 0 256 933
- EP-A1- 0 330 532
- EP-A1- 0 519 144
- EP-A2- 0 122 077
- EP-A2- 0 164 959
- EP-A2- 0 793 958
- WO-A1-82/01649
- WO-A1-96/01621
- FR-A1- 2 722 984

## Description

La présente invention a pour objet une nouvelle composition pharmaceutique présentant une biodisponibilité élevée de par une dissolution supérieure et son procédé de préparation. La présente invention concerne plus particulièrement une composition pharmaceutique destinée à une administration par voie orale, contenant un principe actif de faible solubilité aqueuse.

De nombreux principes actifs ont pour inconvénient de présenter une solubilité faible en milieu aqueux, donc de présenter un profil de dissolution insuffisant et par conséquent une faible biodisponibilité dans l'organisme après administration orale. La dose thérapeutique devant être administrée doit donc être augmentée pour obvier cet inconvénient. C'est le cas notamment de nombreux principes actifs hypolipémiants, tels que ceux appartenant à la famille des fibrates.

Le fénofibrate est un hypolipémiant bien connu de la famille des fibrates qui est commercialisé à divers dosages (100 et 300 mg, par exemple Secalip®), mais sous une forme conduisant à une faible biodisponibilité du principe actif. En effet, du fait de sa faible hydrosolubilité, le fénofibrate est mal absorbé au niveau du tube digestif et présente par conséquent une biodisponibilité incomplète, irrégulière et souvent variable d'un individu à l'autre.

Pour améliorer le profil de dissolution du fénofibrate et sa biodisponibilité et réduire ainsi la dose devant être administrée, il serait utile d'augmenter sa dissolution de manière à ce qu'elle puisse atteindre un niveau proche de 100%.

De plus pour le confort du patient, il est avantageux de rechercher une forme galénique ne nécessitant qu'une seule prise par jour qui permette un effet identique à celui obtenu lors de prises multiples.

Un procédé visant à améliorer la biodisponibilité du fénofibrate est décrit dans le brevet EP-A-0 330 532. Ce brevet décrit l'effet de la co-micronisation du fénofibrate avec un tensio-actif, par exemple du laurylsulfate de sodium pour améliorer la solubilité du fénofibrate et augmenter ainsi sa biodisponibilité. Ce brevet enseigne que la co-micronisation du fénofibrate avec un tensioactif solide permet d'améliorer la biodisponibilité du fénofibrate de façon significativement plus importante que l'amélioration que l'on obtiendrait soit par addition d'un agent tensioactif, soit en micronisant uniquement le fénofibrate, soit encore en mélangeant intimement le fénofibrate et le tensioactif micronisés séparément. La méthode de dissolution utilisée est la technique classique de la palette tournante (Pharmacopée Européenne): la cinétique de dissolution du produit est mesurée dans un volume fixe de milieu de dissolution, agité par un dispositif standardisé; un essai a également été réalisé avec une technique alternative de la Pharmacopée Européenne, à savoir la méthode de la cellule à flux continu.

Ce procédé selon le brevet EP-A-0 330 532 conduit à une nouvelle forme galénique où le produit actif, co-micronisé avec un tensioactif solide, présente une dissolution du fénofibrate améliorée, donc une biodisponibilité augmentée, ce qui permet, à efficacité égale, une diminution de la dose quotidienne de médicament: respectivement 67 mg et 200 mg au lieu de 100 mg et 300 mg.

Cependant, le procédé de préparation selon ce brevet n'est pas totalement satisfaisant dans la mesure où il ne conduit pas à une biodisponibilité complète du principe actif et il présente plusieurs inconvénients. La technique de co-micronisation du fénofibrate avec un tensio-actif solide améliore certes la dissolution de ce principe actif, mais cette dissolution reste incomplète.

Il existe donc un besoin pour améliorer la biodisponibilité du fénofibrate afin d'atteindre, dans des temps très courts, un niveau proche de 100% (ou, en tout cas, supérieur aux limites suivantes : 10% en 5 minutes, 20% en 10 minutes, 50% en 20 minutes et 75% en 30 minutes dans un milieu constitué de 1200 ml d'eau additionnée de 2% de Polysorbate 80 ou de 1000 ml d'eau additionnée de lauryl sulfate de sodium 0,025 molaire, avec une vitesse de rotation de la palette de 75 t/min), et ce même lorsque des milieux de dissolution à faible teneur en tensioactif sont utilisés.

La demanderesse a mis en évidence de façon surprenante qu'il est possible de résoudre ce problème par un nouveau procédé de préparation d'une composition pharmaceutique par pulvérisation d'une suspension du principe actif sur un support inerte hydrosoluble. La présente invention concerne également les compositions pharmaceutiques ainsi préparées.

On connaît déjà l'utilisation de polymère tel que la polyvinylpyrrolidone pour la fabrication de comprimés, à des concentrations de l'ordre de 0,5 à 5% en poids, au maximum de 10% en poids. Dans ce cas, la polyvinylpyrrolidone est utilisée comme liant. De même, on connaît l'utilisation de polymère tel que l'hydroxyméthylpropylméthylcellulose comme liant de granulation. Ainsi, EP-A-0 519 144 décrit des pellets d'une substance faiblement soluble, l'oméprazole, qui sont obtenus par pulvérisation sur des pellets inertes, dans un granulateur à lit fluidisé, d'une dispersion ou suspension de principe actif dans une solution contenant ledit polymère. Cependant, là encore, le polymère (HPMC et HPC) n'est utilisé qu'en tant que liant de granulation, en une quantité d'environ 50% en poids du poids du principe actif, ce qui compte tenu de la présence des pellets inertes de grande taille (environ 700µm) et de la quantité totale finale conduit à des teneurs finales en principe actif et en polymère très faibles, de l'ordre de quelque % à peine du poids du pellet final recouvert. Enfin, on remarquera que la dimension des pellets inertes dans ce document est assez élevée, ce qui dans le cas du fénofibrate conduirait à un volume final de la formulation beaucoup trop grand pour une administration aisée par voie orale.

On connaît aussi l'utilisation de polymère tel que la polyvinylpyrrolidone pour la fabrication de "dispersions solides", obtenues en général par co-précipitation, co-fusion ou mélange en phase liquide suivie d'un séchage. Il s'agit dans ce cas d'une fixation du principe actif en microparticules isolées sur la polyvinylpyrrolidone, ce qui évite les problèmes de mauvais mouillage du solide et de réagglomération des particules. L'article "Stable Solid Dispersion System Against Humidity", par Kuchiki et al, Yakuzaigaku, 44, No.1, 31-37 (1984) décrit une telle technique de préparation de dispersions solides utilisant de la polyvinylpyrrolidone. Les quantités de PVP sont alors ici très importantes, et les rapports principe actif sur PVP sont compris entre 1/1 et 1/20. Dans ce cas cependant, il n'y a pas de support inerte.

On connaît encore d'après le document WO-A-96 01621 une composition à effet retard, comprenant un noyau inerte (silice dans tous les exemples) revêtu d'une couche comprenant le principe actif en mélange avec un polymère hydrophile, le rapport pondéral principe actif/polymère étant compris entre 10/1 et 1/2 et le rapport pondéral principe actif/noyau inerte étant compris entre 5/1 et 1/2, avec une couche externe pour conférer l'effet retard. Ces compositions peuvent être comprimées. Le polymère hydrophile peut être de la polyvinylpyrrolidone. Ce document décrit aussi un procédé .de préparation de cette composition; par exemple dans un granulateur à lit fluidisé, on pulvérise une dispersion de principe actif dans une solution de polymère sur des noyaux inertes. Ce document n'a trait qu'à des compositions à effet retard, le problème technique à résoudre selon ce document étant la compression sans dommage pour la couche externe conférant l'effet retard.

EP-A-0 256 933 décrit un granule obtenu par applications successives de couches de fénofibrate et de couches humides et collantes, les couches collantes comprenant un polymère.

Cependant, rien dans l'état de la technique n'enseigne ni ne suggère la présente invention.

Ainsi, la présente invention décrit une composition de fénofibrate à libération immédiate comprenant:
(a) un support inerte hydrosoluble recouvert d'au moins une couche contenant du fénofibrate sous forme micronisée avec une taille inférieure à 20 µm, un polymère hydrophile et éventuellement un tensio-actif; ledit polymère hydrophile représentant au moins 20% en poids du poids de l'élément a); et
(b) éventuellement une ou plusieurs phase(s) ou couche(s) externe(s).

Selon un mode de réalisation, un tensio-actif est présent avec le fénofibrate et le polymère hydrophile.

L'invention décrit aussi une composition comprenant du fénofibrate présentant une dissolution d'au moins 10% en 5 minutes, 20% en 10 minutes, 50% en 20 minutes et 75% en 30 minutes, telle que mesurée conformément à la méthode de la palette tournante à 75 t/min selon la Pharmacopée Européenne, dans un milieu de dissolution constitué d'eau avec 2% en poids de polysorbate 80 ou un milieu de dissolution constitué d'eau avec 0,025 M de laurylsulfate de sodium.

L'invention à pour objet un procédé de préparation d'un granulé comprenant la pulvérisation sur un support inerte d'une suspension de fénofibrate micronisé avec une taille inférieur à 20 µm dans une solution de polymère hydrophile et éventuellement de tensio-actif. Des modes de réalisation font l'objet des revendications 2 à 26. L'invention à aussi pour objet le granulé susceptible d'être obtenu par le procédé selon l'invention, objet de la revendication 27. Des modes de réalisation font l'objet des revendications 28 à 30.

L'invention décrit une suspension de fénofibrate sous forme micronisée avec une taille inférieure à 20 µm, dans une solution de polymère hydrophile et éventuellement de tensio-actif.

La présente invention est décrite plus en détail dans la description qui suit, en référence aux dessins annexés, dans lesquels:
- la figure 1 est une représentation graphique d'une étude comparative du profil de dissolution d'une composition selon la présente invention et de celui du Lipanthyl® 200 M;
- la figure 2 est une représentation graphique d'une étude comparative du profil de dissolution d'une composition selon la présente invention et de celui de produits pharmaceutiques disponibles sur le marché allemand;

On entend, dans le cadre de la présente invention, par l'expression "sous forme micronisée" une substance se trouvant sous une forme particulaire, la dimension des particules étant inférieure ou égale à environ 20 µm.

Avantageusement, cette dimension est inférieure ou égale à 10µm.

On entend, dans le cadre de la présente invention par "support inerte hydrosoluble" tout excipient, généralement hydrophile, pharmaceutiquement inerte, cristallin ou amorphe, sous une forme particulaire, ne conduisant pas à une réaction chimique dans les conditions opératoires utilisées, et qui est soluble dans un milieu aqueux, notamment en milieu acide gastrique. Des exemples de tels excipients sont les dérivés de sucres, tels que lactose, saccharose, de l'amidon hydrolysé (malto-dextrine), etc.. Des mélanges sont aussi appropriés. La dimension particulaire unitaire du support inerte hydrosoluble peut être par exemple comprise entre 50 et 500 microns.

On entend, dans le cadre de la présente invention par "polymère hydrophile" toute substance de poids moléculaire élevé, (par exemple supérieur à 300) ayant une affinité suffisante pour l'eau pour s'y dissoudre ou y former un gel. Des exemples de tels polymères sont : polyvinylpyrrolidone, poly(alcool vinylique), hydroxypropylcellulose, hydroxyméthylcellulose, hydroxypropylméthylcellulose, gélatine, etc.. Des mélanges de polymères sont aussi appropriés.

Le polymère hydrophile préféré est la polyvinylpyrrolidone (PVP). La PVP utilisée dans le cadre de la présente invention présente par exemple un poids moléculaire compris entre 10 000 et 100 000, de préférence par exemple entre 20 000 et 55 000.

Le terme "tensio-actif" tel qu'utilisé dans le cadre de la présente invention est utilisé dans son sens classique. Tout tensio-actif peut être utilisé, qu'il soit amphotère, non-ionique, cationique ou anionique. Des exemples de tels tensio-actifs sont : sodium lauryl sulfate, monooléate, monolaurate, monopalmitate, monostéarate ou un autre ester de sorbitanne polyoxyéthyléné, dioctylsulfosuccinate de sodium (DOSS), lécithine, alcool stéarylique, alcool cétostéarylique, cholestérol, huile de ricin polyoxyéthylénée, glycérides d'acides gras polyoxyéthylénés, poloxamero, etc.. Des mélanges de tensio-actifs sont aussi appropriés.

Le tensio-actif préféré est le laurylsulfate de sodium, qui peut être co-micronisé avec le fénofibrate.

Les compositions selon l'invention peuvent en outre contenir tout excipient classiquement utilisé dans le domaine pharmaceutique et chimiquement compatible avec le principe actif, tels que les agents liants, les charges, les pigments, les agents de désintégration, les lubrifiants, les agents mouillants, les tampons, etc. On peut citer à titre d'exemple de tels excipients utilisables dans la présente invention: cellulose microcristalline, lactose, amidon, silice colloïdale, talc, esters de glycérol, stéaryl fumarate de sodium, dioxyde de titane, stéarate de magnésium, acide stéarique, polyvinyl pyrrolidone réticulée (AC DI SOL®), carboxyméthylamidon (Explotab®, Primojel®), hydroxypropyl-cellulose, hydroxyméthylcellulose, hydroxypropylméthylcellulose, gélatine, etc..

On entend par "phase ou couche externe" dans le cadre de la présente invention tout revêtement sur l'élément (a) avec le principe actif (formant un "noyau"). En effet, il peut être intéressant de disposer une ou plusieurs phase(s) ou couche(s) au-dessus du noyau revêtu. L'invention couvre ainsi un noyau unique avec une couche, mais aussi plusieurs noyaux dans une phase comme dans le cas de comprimés formés à partir de "noyaux" mélangés avec une phase. Par "phase ou couche externe" dans le cadre de la présente invention, on n'entend pas les révêtements conférant un effet retard à la composition.

Cette couche externe comprend des excipients classiques.
On peut aussi disposer une couche comprenant des adjuvants pour la fabrication de comprimés. Selon ce mode de réalisation, la couche externe comprend un agent de désintégration et par exemple un lubrifiant; les granulés ainsi recouverts et mélangés peuvent alors être facilement comprimés et se désintègrent facilement dans l'eau.

Les compositions selon la présente invention comprennent en général, par rapport au poids total de la composition hors phase ou couche externe, un support inerte hydrosoluble représentant de 10 à 80% en poids, de préférence 20 à 50% en poids, le fénofibrate représentant de 5 à 50% en poids, de préférence 20 à 45% en poids, le polymère hydrophile représentant de 20 à 60% en poids, de préférence 25 à 45% en poids, le tensio-actif représentant de 0 à 10% en poids, de préférence 0,1 à 3% en poids.

La couche ou phase externe, s'il y en a une, peut représenter jusqu'à 80% en poids du poids total, de préférence jusqu'à 50% en poids.

Le polymère hydrophile est présent de préférence en plus de 25% en poids, par rapport au poids de l'élément a).

Le rapport pondéral fénofibrate/polymère hydrophile peut être compris, par exemple entre 1/10 et 4/1, de préférence par exemple entre 1/2 et 2/1.

Quand un tensio-actif est utilisé, le rapport pondéral tensio-actif/polymère hydrophile peut être compris, par exemple entre 1/500 et 1/10, de préférence par exemple entre 1/100 et 5/100.

Selon un mode de réalisation, la composition selon la présente invention se présente sous la forme de comprimés.

Ce comprimé résulte avantageusement de la compression d'éléments (a) (sous forme de granulés) avec une phase externe.

Selon un autre mode de réalisation, la composition selon la présente invention se présente sous la forme de granulés enfermés dans une gélule, par exemple de gélatine, ou dans un sachet.

Les compositions selon la présente invention sont particulièrement appropriées pour l'administration par voie orale des principes actifs.

La composition selon la présente invention est préparée par un nouveau procédé comprenant la pulvérisation sur les noyaux inertes d'une suspension de principe actif sous forme micronisée dans une solution d'un polymère hydrophile et éventuellement de tensio-actif.

Lorsqu'un tensio-actif est présent, le principe actif peut être co-micronisé avec le tensio-actif. On utilise avec avantage la technique selon le document EP-A-0 330 532.

Le procédé selon l'invention consiste à utiliser le principe de la technique de granulation en lit fluidisé, mais avec des produits de départ spécifiques, afin d'aboutir à un profil de dissolution amélioré et ainsi à une biodisponibilité élevée. En particulier, l'invention fait emploi d'une suspension du principe actif micronisé dans une solution d'un polymère hydrophile et éventuellement d'un tensio-actif.

La technique de granulation en lit fluidisé est largement utilisée dans l'industrie pharmaceutique pour préparer des gélules ou des comprimés. De façon classique selon l'art antérieur, une poudre ou un mélange de poudres (principe actif + excipients) est mis en suspension en lit fluidisé dans le granulateur, et une solution contenant un liant et éventuellement un tensio-actif est pulvérisée sur ce lit pour former des granulés. La technique de granulation en lit fluidisé est bien connue de l'homme de l'art qui se rapportera aux ouvrages de référence, par exemple à l'ouvrage "Die Tablette", de Ritschel, Ed. Cantor Aulendorf, pages 211-212.

L'invention, comme il a été indiqué, comprend la pulvérisation sur un support inerte, d'une suspension de principe actif micronisé avec un polymère hydrophile. A l'issue de la granulation, le granulé qui est formé est constitué de cristaux par ex. de lactose, isolés (ou éventuellement agglomérés entre eux par la solution de pulvérisation), et des particules de principe actif et de PVP collés à la surface des cristaux. Le granulé pourrait de même être constitué de cristaux revêtus agglomérés entre eux, voire même d'un tel agglomérat à nouveau revêtu.

Les compositions selon l'invention peuvent aussi être préparées par d'autres procédés, par exemple par pulvérisation de la solution de principe actif micronisé sur le support inerte hydrosoluble.

Les granulés ainsi obtenus peuvent, si cela est souhaité, être enrobé d'une couche externe ou compacté en des comprimés ou former des agglomérats.

La ou les couche(s) externe(s) est(sont) appliquée(s) par des techniques de revêtement classiques, telles que par revêtement dans une cuve ou en lit fluidisé.

Lorsque le granulé obtenu (ultérieurement revêtu ou non) est compacté pour former des comprimés, cette étape peut être mise en oeuvre par toute technique classique appropriée, par exemple sur machine à comprimer alternative ou rotative.

Le produit de départ important est la suspension de principe actif. Cette suspension est préparée par mise en suspension du principe actif micronisé dans une solution, comprenant le polymère hydrophile et éventuellement un agent tensioactif en solution dans un solvant. Si un tensio-actif est utilisé, il est mis en solution dans le solvant (bêcher + agitateur magnétique ou agitateur à pales). Ensuite le polymère hydrophile (PVP) est dispersé sous agitation dans la solution précédemment obtenue. Selon la solubilité du polymère, celui-ci se dissout dans la solution ou forme un gel ou une suspension plus ou moins épais(se). Sous agitation toujours, le principe actif micronisé est dispersé en pluie dans la solution ou suspension précédente pour former une suspension homogène. On peut intervertir l'ordre de ces étapes. Le solvant utilisé peut être aqueux ou organique (par exemple éthanol). On utilise par exemple de l'eau déminéralisée.
La concentration en principe actif dans la suspension est de 1 à 40% en poids, de préférence 10 à 25%.
La concentration en polymère hydrophile dans la suspension est de 5 à 40% en poids, de préférence 10 à 25%.
La concentration en tensio-actif dans la suspension est de 0 à 10% en poids, de préférence inférieure à 5%.

L'invention a aussi pour objet cette nouvelle suspension.

Sans vouloir être liée par une théorie, la demanderesse pense que ce nouveau procédé, par l'utilisation d'une suspension du principe actif micronisé dans une solution de polymère hydrophile, permet l'obtention d'une composition nouvelle dans laquelle le principe actif est sous forme non-réagglomérée.

Les exemples suivants illustrent l'invention sans la limiter.

### Exemple 1 Préparation d'une composition pharmaceutique de fénofibrate selon l'invention.

On prépare une composition contenant en tant qu'élément a) du fénofibrate micronisé, de la Plasdone®, du Capsulac® et du lauryl sulfate de sodium.

Le fénofibrate micronisé présente une dimension particulaire d'environ 5µm, telle que mesurée à l'aide d'un compteur Coulter.

Le Plasdone K25® correspond à.une polyvinylpyrrolidone PVP ISP et le Capsulac 60® (MEGGLE) correspond à un lactose monohydrate à gros cristaux (taille de particules entre 100 et 400 µm).

Le laurylsulfate de sodium (7g) est dissous dans l'eau (eau déminéralisée, 1750g) et le fénofibrate micronisé (350g) est mis en suspension dans le mélange obtenu (par exemple à l'aide d'un agitateur à hélice à 300 t/min, pendant 10 minutes, puis à l'aide d'un agitateur Ultra Turrax à 10 000 t/min, pendant 10 minutes). On ajoute ensuite sous agitation la PVP (350g), l'agitation (agitateur à hélice) étant poursuivie jusqu'à dissolution de cette dernière (30 minutes). L'ensemble est passé sur un tamis (taille 350 µm) pour éliminer d'éventuels agglomérats.

Séparément, le lactose (400g) est mis en suspension dans un granulateur en lit d'air fluidisé (type Glatt® GPCG1 - Top Spray ou équivalent) et on le porte à une température de 40°C.

La suspension de fénofibrate est pulvérisée sur le lactose. Cette étape est réalisée dans les conditions suivantes: pression de pulvérisation: 2,1 bar; débit d'air 70 m³/h, température d'arrivée d'air: 45°C; température de sortie d'air: 33°C; température produit: 34°C; durée de pulvérisation: 3 h.

Le granulé ainsi obtenu peut être mis en gélules ou transformé en comprimés. Toute technique classique appropriée de préparation de telles formulations galéniques peut être utilisée.

Pour la transformation en comprimés, on ajoute à 191g de granulés obtenus (par exemple à l'aide d'un mélangeur type mélangeur-malaxeur, mélangeur planétaire, ou mélangeur par retournement) la phase externe présentant la composition suivante:
- 56g de Polyplasdone XL® (polyvinylpyrrolidone réticulée, ISP, telle que décrite dans la pharmacopée US "USP - NF" sous le nom de crospovidone, MW moyen > 1000000);
- 88g d'Avicel® PH200 (Cellulose microcristalline);
- 3,5g de stéaryl fumarate de sodium (Mendell, U.S.A.); et
- 2g d'Aerosil® 200 (silice colloïdale).

La polyvinylpyrrolidone réticulée, la cellulose microcristalline, le stéaryl fumarate de sodium et la silice colloïdale sont des agents respectivement de désintégration, liant, lubrifiant et d'écoulement.

L'obtention du comprimé peut s'effectuer sur une machine à comprimer alternative (par exemple Korsch EKO) ou rotative (par ex. Fette Perfecta 2).

On obtient ainsi des comprimés présentant la composition suivante, exprimée en mg:

| - élément (a) : | |
|---|---|
| Fénofibrate micronisé | 100,0 |
| PVP | 100,0 |
| Lactose | 114,3 |
| Laurylsulfate de sodium | 2,0 |

| - phase (ou couche) externe : | |
|---|---|
| PVP réticulée | 92,7 |
| Cellulose microcristalline | 145,7 |
| Stéryl fumarate de sodium | 5,8 |
| Silice colloïdale | 3,3 |

### Exemple 2: Dissolution d'une composition selon l'invention et d'une composition selon l'art antérieur.

### a) milieu de dissolution et protocole pour la mesure de la dissolution.

On recherche un milieu de dissolution qui soit discriminant, c'est-à-dire que deux produits ayant des profils de dissolution très différents dans le suc gastrique présenteront des courbes de dissolution très différentes.

On utilise à cette fin un milieu aqueux contenant un tensio-actif, à savoir le Polysorbate 80 (mono oléate de sorbitanne polyoxyéthyléné). Ce tensio-actif est facilement disponible auprès de plusieurs fournisseurs, fait l'objet d'une monographie dans les pharmacopées, et est aisé à mettre en oeuvre (produit liquide soluble dans l'eau). D'autres tensioactifs comme le lauryl sulfate de sodium peuvent également être utilisés.

On utilise la méthode de la palette tournante (Pharmacopée Européenne) dans les conditions suivantes: volume du milieu: 1200 ml; température du milieu: 37°C; vitesse de rotation de la palette: 75 t/min; prélèvements: toutes les 2,5 minutes. La détermination de la quantité dissoute est effectuée par spectrophotométrie. Les essais sont répétés à 6 reprises.

### b) résultats.

La composition selon l'invention consiste en deux comprimés dosés à 100 mg de fénofibrate environ, préparés selon l'exemple 1.

La composition selon l'art antérieur est du Lipanthyl ® 200 M de Laboratoires Fournier, dosé à 200 mg de fénofibrate (correspondant à des gélules de 200 mg de fénofibrate co-micronisé avec du laurylsulfate de sodium, et renfermant du lactose, de l'amidon prégélatinisé de la polyvinylpyrrolidone réticulée et du stéarate de magnésium, conformément à l'enseignement du brevet EP-A-0 330 532).

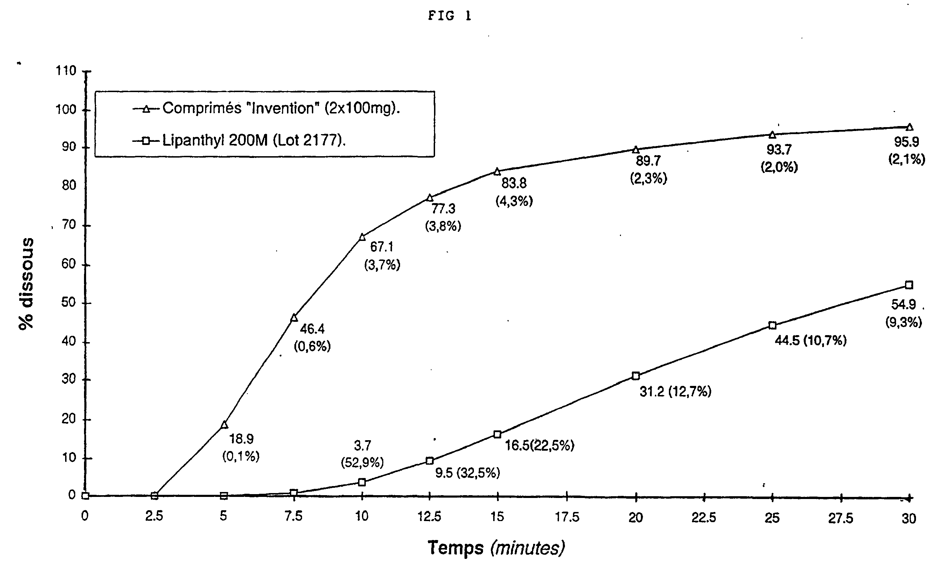

Les résultats obtenus sont représentés graphiquement à la figure 1, sur laquelle sont indiqués le pourcentage de dissolution et entre parenthèses l'écart type observé.

Ces résultats montrent clairement que les compositions selon la présente invention présentent un profil de dissolution nettement supérieur à celui des compositions selon l'art antérieur.

Ces résultats montrent aussi clairement qu'avec les compositions selon l'invention, l'écart type observé est nettement plus faible qu'avec les compositions selon l'art antérieur.

### Exemple 3: Etude de la biodisponibilité des compositions selon la présente invention et de compositions selon l'art antérieur.

Un essai de biodisponibilité sur volontaires sains a été mené.

Les compositions testées sont les suivantes :
- composition selon l'invention: des gélules contenant les granulés préparés selon l'exemple 1, et dosées à 200 mg de fénofibrate.
- première composition selon l'art antérieur: Lipanthyl ® 200 M de Laboratoires Fournier, dosé à 200 mg de fénofibrate, identique à celle de l'exemple précédent.
- seconde composition selon l'art antérieur: Secalip ® en gélules (300 mg de fénofibrate sous forme de 3 gélules à 100mg).

L'étude a été réalisée sur 6 volontaires sains recevant une dose unique de fénofibrate, avec une période de repos de 6 jours minimum entre les administrations. Les échantillons pour analyse pharmacocinétique sont recueillis après chaque administration au temps : 0,5; 1 h; 2 h; 3 h; 4 h; 5 h; 6 h; 8 h; 10 h; 12 h; 24 h; 36 h; 48 h; 72 h et 96 heures après la prise du médicament. La teneur en acide fénofibrique dans le plasma est mesurée sur chaque échantillon.

Les résultats obtenus sont donnés dans le tableau 1 ci-dessous.

**Tableau 1**

| Produit | Dose (mg) | Cmax (µg/ml) | tmax (h) | t1/2 (h) | AUC 0-t (µg.h/ml) | AUC 0 - ∞ (µg.h/ml) |
|---|---|---|---|---|---|---|
| Invention | 200 | 5,4 | 6 | 23 | 148 | 162 |
| Secalip® 100 | 3 x 100 | 1,1 | 25 | 39 | 53 | 56 |
| Lipanthyl® 200M | 200 | 1,6 | 8,3 | 41 | 71 | 92 |

| | | | | | | |
|---|---|---|---|---|---|---|
| Cmax: Concentration plasmatique maximale | | | | | | |
| tmax: temps nécessaire pour atteindre le Cmax | | | | | | |
| t1/2: Demi vie plasmatique | | | | | | |
| AUC 0- t: Aire Sous la Courbe de 0 à t | | | | | | |
| AUC 0 - ∞: Aire Sous la Courbe de 0 à l' ∞ | | | | | | |

Ces résultats montrent clairement que les compositions selon la présente invention, présentant un profil de dissolution amélioré par rapport aux compositions de l'art antérieur, conduisent à une biodisponibilité du principe actif qui est nettement supérieure à celle obtenue dans le cas des compositions selon l'art antérieur.

### Exemple 4 Comparaison du profil de dissolution des compositions selon l'invention avec celui de produits actuellement sur le marché en Allemagne

Sur le marché allemand on trouve des formulations de fénofibrate à action immédiate ou à action prolongée. Comme en France, les formes à 100 & 300 mg (classiques) coexistent avec des formes à 67 et 200 mg (à biodisponibilité améliorée, selon l'enseignement du brevet EP-A-0 330 532).

Ces produits sont les suivants:
- Fénofibrate - Ratiopharm; Ratiopharm - Ulm; Gélules;
   Composition: Fénofibrate 100 mg;
   Excipients: Lactose, amidon de maïs, stéarate de magnésium, colorant E 171, gélatine.
- Durafenat; Durachemie - Wolfrathausen; Gélules;
   Composition: Fénofibrate 100 mg;
   Excipient: Lactose, amidon de maïs, stéarate de magnésium, colorant E 171, gélatine.
- Normalip pro; Knoll - Ludwigshaffen; Gélules;
   Composition: Fénofibrate 200 mg;
   Excipients: Crospovidone, gélatine, lactose monohydrate, stéarate de magnésium, amidon de maïs, laurylsulfate de sodium, colorants E 132 et E 171.

On effectue une comparaison entre:
- le comprimé selon l'invention tel que préparé selon l'exemple 1 (2 x 100 mg) ;
- le Normalip pro ® (200 mg);
- le Lipanthyl ® 200 M (200 mg) (selon l'exemple précédent);
- le Fénofibrate Ratiopharm® (2 x 100 mg);
- le Durafenat ® (2 x 100 mg).

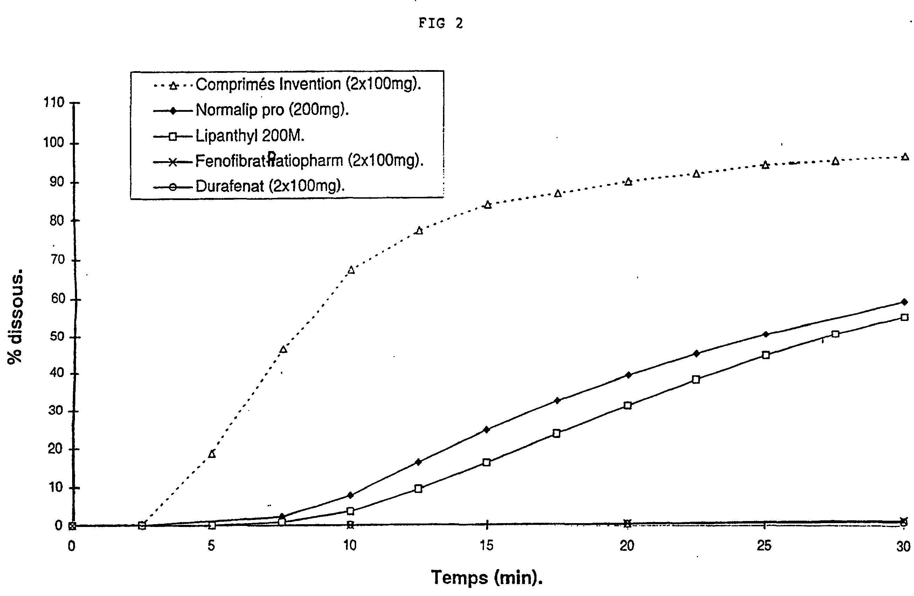

Les tests sont mis en oeuvre dans les mêmes conditions que dans les exemples précédents. Les résultats sont reportés figure 2.

Ces résultats montrent clairement que les compositions selon l'invention présentent une dissolution nettement améliorée par rapport aux compositions selon l'art antérieur.

## Revendications

1. Procédé de préparation d'un granulé comprenant pulvérisation sur un support inerte d'une suspension de fénofibrate sous forme micronisée avec une taille inférieure à 20 µm, dans une solution de polymère hydrophile et éventuellement de tensio-actif.

2. Procédé selon la revendication 1, lesdits granulés comprenant des particules de support inerte hydrosoluble, isolées ou éventuellement agglomérées entre elles, liées avec des particules de fénofibrate micronisé ayant une taille inférieure à 20 µm en mélange avec un polymère hydrophile adhérant à la surface des particules de support inerte hydrosoluble, les granulés comprenant éventuellement une ou plusieurs phase(s) ou couche(s) externe(s), ou étant éventuellement agglomérés, la quantité de polymère hydrophile représentant au moins 20% en poids.

3. Procédé selon la revendication 1, lesdits granulés comprenant (i) des particules de support inerte hydrosoluble; et (ii) une ou plusieurs couches comprenant du fénofibrate micronisé ayant une taille inférieure à 20 µm dispersé dans un polymère hydrophile, une ou plusieurs couche(s) étant déposées sur les particules de support inerte hydrosoluble, la quantité de polymère hydrophile représentant au moins 20% en poids.

4. Procédé selon la revendication 1, 2 ou 3, dans lequel le fénofibrate a une dimension particulaire inférieure ou égale à 10 µm.

5. Procédé selon l'une des revendications 1 à 4, dans lequel le polymère hydrophile est choisi parmi: polyvinylpyrrolidone, poly(alcool vinylique), hydroxypropylcellulose, hydroxy-méthylcellulose, hydroxypropylméthylcellulose, gélatine et leurs mélanges.

6. Procédé selon la revendication 5, dans lequel le polymère hydrophile est la polyvinylpyrrolidone.

7. Procédé selon l'une des revendications 1 à 6, dans lequel lesupport inerte hydrosoluble est un dérivé de sucre ou de l'amidon hydrolysé ou Leurs mélanges.

8. Procédé selon l'une des revendications 1 à 7, dans lequel le support inerte hydrosoluble est du lactose.

9. Procédé selon l'une des revendications 1 à 8, dans lequel le support inerte hydrosoluble présente une dimension particulaire unitaire comprise entre 50 et 500 microns.

10. Composition selon la revendication 9, dans lequel le support inerte hydrosoluble est du lactose présentant une dimension particulaire unitaire comprise entre 100 et 400 microns.

11. Procédé selon l'une des revendications 1 à 10, dans lequel la suspension comprend de plus un tensio-actif.

12. Procédé selon la revendication 11, dans lequel le tensio-actif est choisi parmi sodium lauryl sulfate, monooléate, monolaurate, monopalmitate, monostéarate ou un autre ester de sorbitanne polyoxyéthyléné, dioctylsulfosuccinate de sodium (DOSS), lécithine, alcool atéarylique, alcool cécastéarylique, cholestérol, huile de ricin polyoxyéthylénée, glycérides d'acides gras polyoxyéthylénés, poloxamer, et leurs mélanges.

13. Procédé selon la revendication 11 ou 12, dans lequel le tensio-actif est du sodium lauryl sulfate.

14. Procédé selon l'une des revendications 11 à 13, dans lequel le fénofibrate et le tensio-actif sont co-micronisés.

15. procédé selon l'une des revendications 1 à 14, dans lequel le fénofibrate des granulés est sous forme non-réagglomérée.

16. Procédé selon l'une quelconque des revendications précédentes comprenant les étapes de:
(a) préparation d'une suspension de fénofibrate sous forme micronisée avec une taille inférieure à 20 µm, dans une solution de polymère hydrophile et éventuellement de tensio-actif;
(b) application de la suspension de l'étape (a) sur un support inerte hydrosoluble;
(c) éventuellement enrobage des granulés ainsi obtenus par une ou plusieurs phase(s) ou couche(s)

17. Procédé selon la revendication 16, dans lequel l'étape (b) est mise en oeuvre dans un granulateur à lit fluidisé.

18. Procédé selon la revendication 16 ou 17, comprenant l'étape de compression des produits obtenus à l'étape (b) ou (c)avec ou sans excipients supplémentaires.

19. Procédé selon l'une des revendications 1 à 15, dans lequel la pulvérisation est mise en oeuvre dans un granulateur à lit fluidisé.

20. Procédé selon l'une des revendications 1 à 15 ou 19, comprenant l'étape de compression des granulés, avec ou sans excipients supplémentaires.

21. Procédé selon l'une des revendications 1 à 20, dans lequel la concentration en fénofibrate dans la suspension est de 1 à 40% en poids.

22. Procédé selon la revendication 21, dans lequel la concentration en fénofibrate dans la suspension est de 1 à 40% en poids, de préférence 10 à 25%.

23. Procédé selon l'une des revendications 1 à 22, dans lequel la concentration en polymère hydrophile dans la suspension est de 5 à 40% en poids.

24. Procédé selon la revendication 23, dans lequel la concentration en polymère hydrophile dans la suspension est de 10 à 25%.

25. Procédé selon l'une des revendications 1 à 22, dans lequel la concentration en tensio-actif dans la suspension est de 0 à 10% en poids.

26. Procédé selon la revendication 25, dans lequel la concentration en tensio-actif dans la suspension est inférieure à 5%.

27. Granulé susceptible d'être obtenu par le procédé selon l'une des revendications 1 à 26, la quantité de polymère hydrophile représentant au moins 20% en poids.

28. Granulé selon la revendication 27 dans une gélule.

29. Granulé selon la revendication 27 sous forme de comprimé.

30. Granulé selon l'une des revendications 27 29, présentant une dissolution d'au moins 10% en 5 minutes, 20% en 10 minutes, 50% en 20 minutes et 75% en 30 minutes, telle que mesurée conformément à la méthode de la palette tournante à 75 t/min selon la Pharmacopée Européenne, dans un milieu de dissolution constitué d'eau avec 2% en poids de polysorbate 80 ou 0,025M de lauryl sulfate de sodium.

## Patentansprüche

1. Verfahren zur Herstellung eines Granulats umfassend das Pulverisieren auf einem inerten Träger einer Suspension von Fenofibrat in mikronisierter Form mit einer Größe kleiner als 20 µm in einer Lösung eines hydrophilen Polymers und gegebenenfalls eines Tensids.

2. Verfahren gemäß Anspruch 1, wobei das Granulat isolierte oder gegebenenfalls untereinander agglomerierte Teilchen eines wasserlöslichen, inerten Trägers umfaßt, die mit den mikronisierten Fenofibrattellchen mit einer Größe kleiner als 20 µm im Gemisch mit einem an der Oberfläche der Teilchen des wasserlösllchen, inerten Trägers haftenden hydrophilen Polymer verbunden sind, wobei das Granulat gegebenenfalls eine oder mehr äußere Phase(n) oder Schicht(en) umfaßt oder gegebenenfalls agglomeriert ist und die Menge des hydrophilen Polymers mindestens 20 Gew.-% beträgt.

3. Verfahren gemäß Anspruch 1, wobei das Granulat (i) Teilchen eines wasserlöslichen, inerten Trägers und (ii) eine oder mehr Schichten umfaßt, die in einem hydrophilen Polymer mikronisiertes Fenofibrat mit einer Größe kleiner als 20 µm umfassen, eine oder mehr Schicht(en) auf den Teilchen eines wasserlöslichen, inerten Trägers aufgebracht sind und die Menge des hydrophilen Polymers mindestens 20 Gew.-% beträgt.

4. Verfahren gemäß Anspruch 1, 2 oder 3, bel dem das Fenofibrat eine Teilchengröße kleiner oder gleich 10 µm aufweist.

5. Verfahren gemäß einem der Ansprüche 1 bis 4, bei dem das hydrophile Polymer aus Polyvinylpyrrolidon, Poly(vinylalkohol), Hydroxypropylcellulose, Hydroxymethylcellulose, Hydroxypropylmethylcellulose, Gelatine und ihren Gemischen ausgewählt ist.

6. Verfahren gemäß Anspruch 5, bei dem das hydrophile Polymer Polyvinylpyrrolidon ist.

7. Verfahren gemäß einem der Ansprüche 1 bis 6, bei dem der wasserlösliche, inerte Träger ein Zuckerderlvat oder hydrolysierte Stärke oder ihre Gemische ist.

8. Verfahren gemäß einem der Ansprüche 1 bis 7, bei dem der wasserlösliche, inerte Träger Lactose ist.

9. Verfahren gemäß einem der Ansprüche 1 bis 8, bel dem der wasserlösliche, inerte Träger eine Einzelteilchengröße zwischen 50 und 500 Mikron zeigt.

10. Zusammensetzung gemäß Anspruch 9, bei der der wasserlösliche, inerte Träger Lactose ist, die eine Einzelteüchengröße zwischen 100 und 400 Mikron zeigt.

11. Verfahren gemäß einem der Ansprüche 1 bis 10, bei dem die Suspension außerdem ein Tensid umfaßt.

12. Verfahren gemäß Anspruch 11, bei dem das Tensid aus Natriumlaurylsulfat, einem Monooleat, Monolaurat, Monopalmitat, Monostearat oder einem anderen Ester von pofyoxyethyleniertem Sorbitan, Natriumdioctylsulfosuccinat (DOSS), Lecithin, Stearylalkohol, Ketostearylalkohol, Cholesterin, polyoxyethylenlertem Rizinusöl, polyoxyethylenlerten Fettsäureglyceriden, Poloxamer und ihren Gemischen ausgewählt ist.

13. Verfahren gemäß Anspruch 11 oder 12, bei dem das Tensid Natriumlaurylsulfat ist.

14. Verfahren gemäß einem der Ansprüche 11 bis 13, bei dem das Fenofibrat und das Tensid gemeinsam mikronisiert werden.

15. Verfahren gemäß einem der Ansprüche 11 bis 14, bei dem sich das Fenofibrat des Granulats in nicht-reagglamerierter Form befindet.

16. Verfahren gemäß einem der vorangehenden Ansprüche, umfassend die Schritte:
(a) der Herstellung einer Suspension von Fenofibrat in mikronisierter Form mit einer Größe kleiner als 20 µm in einer Lösung eines hydrophilen Polymers und gegebenenfalls eines Tensids,
(b) des Auftragens der Suspension aus Schritt (a) auf einen wasserlöslichen, inerten Träger,
(c) gegebenenfalls das Überziehens des so erhaltenen Granulats mit einer oder mehr Phase(n) oder Schicht(en).

17. Verfahren gemäß Anspruch 16, bei dem der Schritt (b) In einem Wirbelschichtgranulator durchgeführt wird.

18. Verfahren gemäß Anspruch 16 und 17, das den Schritt des Verpressens der in Schritt (b) oder (c) erhaltenen Produkte mit oder ohne zusätzliche Arzneimittelträger umfaßt.

19. Verfahren gemäß einem der Ansprüche 1 bis 15, bei dem die Pulverisierung in einem Wirbelschichtgranulator durchgeführt wird.

20. Verfahren gemäß einem der Ansprüche 1 bis 15 oder 19, das den Schritt des Verpressens des Granulats mit oder ohne zusätzliche Arzneimittelträger umfaßt.

21. Verfahren gemäß einem der Ansprüche 1 bis 20, bei dem die Fenofibratkonzentration in der Suspension 1 bis 40 Gew.-% ist.

22. Verfahren gemäß Anspruch 21, bel dem die Fenofibratkonzentration in der Suspension 1 bis 40 Gew.-%, bevorzugt 10 bis 25% ist,

23. Verfahren gemäß einem der Ansprüche 1 bis 22, bei dem die Konzentration des hydrophilen Polymers in der Suspension 5 bis 40 Gew.-% ist.

24. Verfahren gemäß Anspruch 23, bei dem die Konzentration des hydrophilen Polymers In der Suspension 10 bis 25 Gew.-% ist.

25. Verfahren gemäß einem der Ansprüche 1 bis 22, bei dem die Konzentration des Tensids in der Suspension 0 bis 10 Gew.-% ist.

26. Verfahren gemäß Anspruch 25, bei dem die Konzentration des Tensids in der Suspension kleiner als 5 Gew.-% ist.

27. Granulat, erhältlich durch dasiverfahren gemäß einem der Ansprüche 1 bis 26, wobei die Menge des hydrophilen Polymers mindestens 20 Gew.-% beträgt.

28. Granulat gemäß Anspruch 27 in einer Kapsel.

29. Granulat gemäß Anspruch 27 in Tablettenform.

30. Granulat gemäß einem der Ansprüche 27 bis 29, das eine gemäß dem Blattrührerverfahren mit 75 Upm gemäß der Europäischen Pharmakopöe in einem Lösungsmedium, das aus Wasser mit 2 Gew.-% Polysorbat 80 oder 0,025 M Natriumlaurylsulfat besteht, gemessene Löslichkeit von mindestens 10% in 5 Minuten, 20% in 10 Minuten, 50% in 20 Minuten und 75% in 30 Minuten zeigt.

## Claims

1. Method of preparing a granule comprising the spraying onto an inert support of a suspension of fenofibrate in micronized form having a size of less than 20 µm, in a solution of hydrophilic polymer and optiotlally of surfactant.

2. Method according to claim 1, said granules containing particles of hydrosoluble inert support, isolated or optionally agglomerated to each other, linked with micronized particles of fenofbrate having a size of less than 20 µm mixed with a hydrophilic polymer adhering to the surface of the particles of hydrosoluble inert support, the granules optionally containing one or more external phase(s) or layer(s), or being optionally agglomerated, the quantity of hydrophilic polymer representing at least 20% by weight.

3. Method according to claim 1, said granules comprising (i) particles of hydrosoluble inert support; and (ii) one or more layers comprising micronized fenofibrate having a size of less than 20 µm dispersed in a hydrophilic polymer, one or more layer(s) being deposited on the particles of hydrosoluble inert support, the quantity of hydrophilic polymer representing at least 20% by weight.

4. Method according to claim 1, 2 or 3, in which the fenofibrate has a particle size of less than or equal to 10 µm.

5. Method according to one of claims 1 to 4, in which the hydrophilic polymer is chosen from: polyvinylpyrrolidone, poly(vinyl alcohol), hydroxypropyl cellulose, hydroxymethyl cellulose, hydroxypropyl methylcellulose, gelatin and their mixtures.

6. Method according to claim 5, in which the hydrophilic polymer is polyvinylpyrrolidone.

7. Method according to one of claims 1 to 6, in which the hydrosoluble inert support is a derivative of sugar or hydrolyzed starch or their mixtures.

8. Method according to one of claims 1 to 7, in which the hydrosoluble inert support is lactose.

9. Method according to one of claims 1 to 8, in which the hydrosoluble inert support has a unit particle size comprised between 50 and 500 microns.

10. Composition according to claim 9, in which the hydrosoluble inert support is lactose having a unit particle size comprised between 100 and 400 microns.

11. Method according to one of claims 1 to 10, in which the suspension also contains a surfactant.

12. Method according to claim 11, in which the surfactant is chosen from sodium lauryl sulphate, monooleate, monolaurate, monopalmitate, monostearate or another polyoxyethylene sorbitan ester, dioctyl sodium sulphosuccinate (DOSS), lecithin, stearyl alcohol, cetostearyl alcohol, cholesterol, polyoxyethylene castor oil, polyoxyethylene fatty acid glycerides, poloxamer and their mixtures.

13. Method according to claim 11 or 12, in which the surfactant is sodium lauryl sulphate.

14. Method according to one of claims 11 to 13, in which the fenofibrate and the surfactant are comicronized.

15. Method according to one of claims 1 to 14, in which the fenofibrate of the granules is in non-reagglomerated form.

16. Method according to any one of the preceding claims comprising the stages of:
(a) preparation of a suspension of fenofibrate in micronized form having a size of less than 20 µm, in a solution of hydrophilic polymer and optionally of surfactant;
(b) application of the suspension of stage (a) to a hydrosoluble inert support;
(c) optionally coating of the thus obtained granules with one or more phase(s) or layer(s).

17. Method according to claim 16, in which stage (b) is carried out in a fluidized-bed pelletizer.

18. Method according to claim 16 or 17, comprising the stage of compression of the products obtained in stage (b) or (c), with or without additional excipients.

19. Method according to one of claims 1 to 15, in which the spraying is carried out in a fluidized-bed pelletizer.

20. Method according to one of claims 1 to 15 or 19, comprising the stage of compression of the granules, with or without additional excipients.

21. Method according to one of claims 1 to 20, in which the concentration of fenofibrate in the suspension is 1 to 40% by weight.

22. Method according to claim 21, in which the concentration of fenofibrate in the suspension is 1 to 40% by weight, preferably 10 to 25% by weight.

23. Method according to one of claims 1 to 22, in which the concentration of hydrophilic polymer in the suspension is 5 to 40% by weight.

24. Method according to claim 23, in which the concentration of hydrophilic polymer in the suspension is 10 to 25%.

25. Method according to one of claims 1 to 22, in which the concentration of surfactant in the suspension is 0 to 10% by weight.

26. Method according to claim 25, in which the concentration of surfactant in the suspension is less than 5%.

27. Granule which is able to be obtained with the method according to one of claims 1 to 26, the quantity of hydrophilic polymer representing at least 20% by weight.

28. Granule according to claim 27 in a gelatin capsule.

29. Granule according to claim 27 in the form of a tablet.

30. Granule according to one of claims 27 to 29, having a dissolution of at least 10% in 5 minutes, 20% in 10 minutes, 50% in 20 minutes and 75% in 30 minutes, as measured using the method with a blade rotating at 75 rpm according to the European Pharmocopoeia, in a dissolution medium constituted by water with 2% by weight of polysorbate 80 or 0.025M of sodium lauryl sulphate.
